Europäisches Patentamt

European Patent Office

Office européen des brevets

(19)

(11) Veröffentlichungsnummer: **0 332 010**
**A2**

(12) **EUROPÄISCHE PATENTANMELDUNG**

(21) Anmeldenummer: **89103441.5**

(22) Anmeldetag: **28.02.89**

(51) Int. Cl.4: **A61L 15/03 , A61L 15/06**

(30) Priorität: **05.03.88 DE 3807283**

(43) Veröffentlichungstag der Anmeldung:
**13.09.89 Patentblatt 89/37**

(84) Benannte Vertragsstaaten:
**AT CH DE ES FR GB IT LI SE**

(71) Anmelder: **BASF Aktiengesellschaft**
**Carl-Bosch-Strasse 38**
**D-6700 Ludwigshafen(DE)**

(72) Erfinder: **Merkle, Hans-Peter, Prof. Dr.**
**Hartmann-Ibach-Strasse 70**
**D-6000 Frankfurt am Main(DE)**
Erfinder: **Lichtenberger, Rainer**
**Heinrich-Fulda-Weg 19**
**D-6100 Darmstadt-Kranichstein(DE)**
Erfinder: **Wendel, Kurt, Dr.**
**Woehlerstrasse 26**
**D-6700 Ludwigshafen(DE)**
Erfinder: **Spengler, Reinhard, Dr.**
**Comeniusstrasse 6**
**D-6700 Ludwigshafen(DE)**
Erfinder: **Kolter, Karl, Dr.**
**Sachensweg 12**
**D-6703 Limburgerhof(DE)**

(54) **Verfahren zur Herstellung eines Pflasters zur transdermalen Anwendung pharmazeutischer Wirkstoffe.**

(57) Verfahren zur Herstellung eines Pflasters zur transdermalen Anwendung von systemisch wirksamen, lipophilen pharmazeutischen Wirkstoffen mit einem Molekulargewicht unter 1 000, durch Mischen einer wäßrigen Dispersion eines Polymeren mit einer Glastemperatur zwischen -70 und -10° C mit dem Wirkstoff, Beschichten einer Trägerfolie, die gegenüber Wasserdampf und dem Wirkstoff undurchlässig ist, mit der wirkstoffhaltigen Polymerdispersion und Trocknen, wobei ein Wirkstoff, der das Polymer nicht anlöst, und kein flüchtiges organisches Lösungsmittel eingesetzt wird.

EP 0 332 010 A2

## Verfahren zur Herstellung eines Pflasters zur transdermalen Anwendung pharmazeutischer Wirkstoffe

Die Erfindung betrifft ein Herstellungsverfahren für eine lösungsmittelfreie Zubereitung in Form eines selbstklebenden, den Wirkstoff enthaltenden Polymerfilms zur transdermalen Langzeitapplikation systemisch wirksamer, lipophiler pharmazeutischer Wirkstoffe mit einem Molekulargewicht unter 1 000 auf einer für Wirkstoff und Wasserdampf weitgehend undurchlässigen Folie.

Zur transdermalen Langzeitapplikation von Wirkstoffen sind verschiedene mehrschichtig zusammengesetzte Systeme bekannt, die einen Film zur Kontrolle der Arzneistofffreisetzung nutzen. Derartige laminar aufgebaute Produkte sind z.B. in den US-PS 3 995 632, 4 060 084, 3 731 683, 3 742 951 oder 4 031 894 beschrieben und bestehen im allgemeinen aus einer undurchlässigen Stützfolie, welche gleichzeitig Schutz nach außen gewährt, einem Reservoir, welches den Wirkstoff in flüssiger oder fester suspendierter Form enthält, einer darauf aufgebrachten geeigneten Vorrichtung zur Steuerung der Wirkstofffreigabe sowie aus Einrichtungen zur klebenden Fixierung des Systems auf der Haut.

Die Herstellung dieser Systeme ist aufgrund ihres mehrschichtigen Aufbaus aufwendig.

Demgegenüber können pharmazeutische Zubereitungen einfacher in Form eines Polymerfilms hergestellt werden, in den der Wirkstoff inkorporiert ist. So sind aus den DE-A- 2 449 865, 3 204 551, 3 525 767, 3 423 293 und 3 242 837 und den EP-A-169 364, 186 109 und 159 168 Zubereitungen bekannt, die aus in Wasser unlöslichen Polymeren bestehen und prinzipiell für eine Langzeittherapie geeignet sind. Die Verwendung organischer Lösungsmittel und -gemische bei allen genannten Systemen bedingt einen hohen technischen und finanziellen Aufwand zur Vermeidung von Gesundheits- und Umweltschäden bei der Herstellung.

Schließlich beschreiben DE-A- 2 929 500 und DD-A- 217 989 Polymerfilme als Wirkstoffträger zur dermalen Wirkstoffapplikation, deren Herstellung aus wäßrigen Polyacrylatdispersionen die obengenannten Herstellungsprobleme vermeidet. Hierbei wird bei DE-A- 2 920 500 der aus Polyethylacrylat und -ethylmethacrylat-Copolymeren hergestellte Film nachträglich mit einer Lösung des Wirkstoffs in einem geeigneten, organischen Lösungsmittel imprägniert. Gemäß DE-A- 3 204 551 ergeben sich jedoch Nachteile bei dieser Herstellungsmethode durch technische Schwierigkeiten bei der Auftragung der Wirkstofflösung und durch mangelnde Reproduzierbarkeit bei der Herstellung der Arzneiform. Nach der DD-A-217 989 werden Lösungen von Wirkstoffen in mit Wasser mischbaren organischen Lösungsmitteln mit geeigneten wäßrigen Polymerdispersionen gemischt und daraus wirkstoffhaltige Filme hergestellt. Aus Gründen der Dispersionsstabilität ist dieses Verfahren nicht für lipophile Wirkstoffe geeignet, außerdem ergeben sich auch hier teilweise Probleme durch Umweltbelastung bei der Herstellung.

Ziel der Erfindung ist es, ein einfaches, wirtschaftliches und umweltfreundliches Verfahren zur Herstellung einer pharmazeutischen Zubereitung zur transdermalen Anwendung zu entwickeln.

Dieses Ziel wird erreicht durch ein Verfahren zur Herstellung eines Pflasters zur transdermalen Anwendung von systemisch wirksamen, lipophilen pharmazeutischen Wirkstoffen mit einem Molekulargewicht unter 1 000, durch Mischen einer wäßrigen Dispersion eines Polymeren mit einer Glastemperatur zwischen -70 und -10, vorzugsweise -55 bis -25 °C, mit dem Wirkstoff, Beschichten einer Trägerfolie, die gegenüber Wasserdampf und dem Wirkstoff praktisch undurchlässig ist, mit der wirkstoffhaltigen Polymerdispersion und Trocknen, wobei ein Wirkstoff, der das Polymere nicht so stark anlöst, daß dessen Haftfähigkeit leidet, und kein flüchtiges organisches Lösungsmittel eingesetzt wird.

Geeignete pharmazeutische Wirkstoffe mit Molekulargewichten unter 1 000 sind lipophiler Natur, so daß sie in der Lage sind, durch die menschliche Haut zu penetrieren. Außerdem dürfen sie die Polymerschicht, in die sie eingebettet sind, nicht anlösen und dadurch das Haftvermögen auf der Haut beeinträchtigen, wie das z.B. bei Nitroglycerin und ähnlichen nitrierten Polyolen der Fall wäre.

Als Beispiele für Arzneistoffklassen mit geeigneten Wirkstoffen seien genannt: Calciumantagonisten, Antihypertensiva, Antiarrhythmika, β-Rezeptorenblocker, starke Analgetika (Opiate), Psychopharmaka, Vasodilatatoren, Antiparkinsonmittel, Anticholinergika, Antihistaminika, Antirheumatika und Hormone. Beispiele für geeignete Wirkstoffe sind: Verapamil, Gallopamil, Anipamil, Hydromorphon, Biperiden, Soquinolol, Nesapidil, Estradiol, Clonidin, Scopolamin, Haloperidol, Bupranolol, Timolol, Fentanyl, Testosteron.

Die Herstellung von klebrigen Polymeren, die ihrerseits zur Herstellung von selbstklebenden Bändern und Folien geeignet sind, ist bekannt, beispielsweise aus den US-Patenten 2 973 286 und 3 307 544. Die selbstklebenden Bänder und Folien sollen auf bloßen Kontakt auf der menschlichen Haut kleben, doch soll die Kohäsion der Klebschicht größer sein als die Adhäsion an der Haut, so daß sie sich weitgehend rückstandslos wieder abziehen lassen. Es handelt sich in der Regel um Copolymerisate auf der Basis von Acryl- und Methacrylsäureestern von Alkoholen mit 2 bis 12, vorzugsweise 4 bis 8 Kohlenstoffatomen, die zahlreiche andere Comonomere einpolymerisiert enthalten können, beispielsweise (Meth-)acrylsäure, (Meth- )acrylnitril, (Meth-)acryl-amid, N-tert.-Butyl-(meth-)acrylamid, Vinylester wie Vinylacetat, -propionat oder -butyrat, andere Vinylverbindungen wie Styrol, ferner Butadien. Besonders hervorgehoben seien Butylacrylat und 2-Ethylhexylacrylat. Die Polymeren können auch durch Zusatz geringer Mengen Comonomerer mit zwei oder mehr copolymerisierbaren Doppelbindungen, also beispielsweise von Diacrylaten, wie Butandioldiacrylat, oder Divinylverbindungen, wie Divinylbenzol, vernetzt sein. Die Teilchengröße der Dispersionen soll zwischen 50 und 500 nm, bevorzugt zwischen 50 und 200 nm liegen. Die Teilchengröße und der Vernetzungsgrad können in bekannter Weise in Abhängigkeit von den Polymerisationsbedingungen und den Comonomeren eingestellt werden. Kleinere Teilchengrößen und ein erhöhter Vernetzungsgrad können eine Steigerung der Wirkstofffreisetzung bewirken.

Die Herstellung der erfindungsgemäßen pharmazeutischen Zubereitung erfolgt in der Weise, daß ein lipophiler Wirkstoff in ungeladener Form mittels eines Emulsionsverfahrens in die wäßrige Latexdispersion eingearbeitet wird. Dazu kann der feinpulvrige (Teilchengröße unter 200, vorzugsweise unter 50 μm) oder flüssige lipophile Wirkstoff in einer wäßrigen Emulgatorlösung dispergiert und in diese Mischung eine der obengenannten wäßrigen Latexdispersionen bei einer Temperatur von 10-80°C, bevorzugt 30-70°C, eingerührt werden. Daneben kann auch das Salz eines sauren oder basischen Wirkstoffes in wäßriger Lösung mit der Polymerdispersion bei einem pH-Wert gemischt werden, bei dem der Wirkstoff vorwiegend in der wasserlöslichen ionisierten Form vorliegt. Durch pH-Verschiebung wird dann der Wirkstoff in die ungeladene wasserunlösliche Form gebracht und simultan in die Dispersion einemulgiert.

Gleichzeitig können mittels dieses Verfahrens zusammen mit dem Wirkstoff auch Schleppmittel eingearbeitet werden, die in der Regel lipophile nichtflüchtige, flüssige Stoffe darstellen, z.B. Isopropylmyristat, N,N-Diethyltoluamid, N,N-Dimethylacetamid, Ethylcaprylat, Azone, Dodecanol. Die Einarbeitung eines solchen Schleppmittels kann dadurch erfolgen, daß dieses entweder mit dem Wirkstoff gemischt, emulgiert und anschließend mit der Polymerdispersion vereinigt wird, oder daß es zeitlich getrennt in eine wäßrige Wirkstoffemulsion bzw. -dispersion eingearbeitet und anschließend mit der Polymerdispersion vereinigt wird.

In jedem Fall legt man zweckmäßig den Wirkstoff vor, gibt den Emulgator und Wasser zu und mischt dann mit der Polymerdispersion. Die so erhaltene wirkstoffhaltige Dispersion wird gegebenenfalls mit üblichen Hilfsstoffen versehen und in an sich bekannter Weise auf einer Stützfolie zu einem Film ausgezogen und getrocknet. Die Trocknungstemperatur kann hierbei zwischen Raumtemperatur und 100°C liegen, wobei ein Optimum zwischen angestrebter schneller Trocknung und zu vermeidender Blasenbildung im Film sowie thermischer Belastung des Wirkstoffs im allgemeinen bei 35-45°C liegt. Ein entscheidender Vorteil gegenüber bestehenden Methoden ist die Vermeidung von organischen Lösungsmitteln bei der Herstellung der Arzneiform.

Die resultierenden Filme haben Dicken von 10-800, bevorzugt 50-300 μm. Die Filmherstellung kann kontinuierlich oder diskontinuierlich erfolgen. Das Auftrageverfahren kann mehrmals wiederholt werden, bis der Film die gewünschte Dicke erreicht hat. Die klebrige Polymerschicht enthält den lipophilen Wirkstoff in einer Konzentration im Bereich von 1 bis 40, vorzugsweise 5 bis 25 Gew.%.

Als Emulgatoren sowohl für die Wirkstoffe wie auch die Polymeren werden die hierfür üblichen Tenside verwendet, wie das Natriumsalz von längerkettigen Fettsäuren und des Schwefelsäurehalbesters eines (ggf. oxethylierten) Fettalkohols als Beispiele für anionische Tenside sowie polyoxethylierte Alkylphenole und längerkettige Fettalkohole (z.B. Hexadekan-(1)-ol) und Glycerin-Fettsäurepartialester als Beispiele für nichtionische Tenside und Coemulgatoren.

Weitere in Betracht kommende Hilfsstoffe sind z.B. Polyacrylsäuren zur Verdickung der wäßrigen Dispersionen sowie z.B. Polyole wie Glycerin und Polyethylenglykol als Weichmacher zur Beeinflussung der Haftfähigkeit der getrockneten Klebeschicht, ferner Quellstoffe, die das Quellungsvermögen des Filmes verbessern und dadurch die Freisetzung des Wirkstoffs beschleunigen, z.B. Cellulosederivate, Polyacrylate, Polyvinylpyrrolidon.

Die Stützfolie, auf die die wirkstoffhaltige Polymerdispersion aufgetrocknet wird, soll sowohl für den Wirkstoff wie für Wasserdampf praktisch undurchlässig sein. Sie kann z.B. aus einer Aluminium-Kunststoff-Verbundfolie oder einer Kunststofolie, die zur Wirkstoffseite hin mit einer Sperrschicht aus z.B. Polyvinylidenchlorid versehen ist, bestehen.

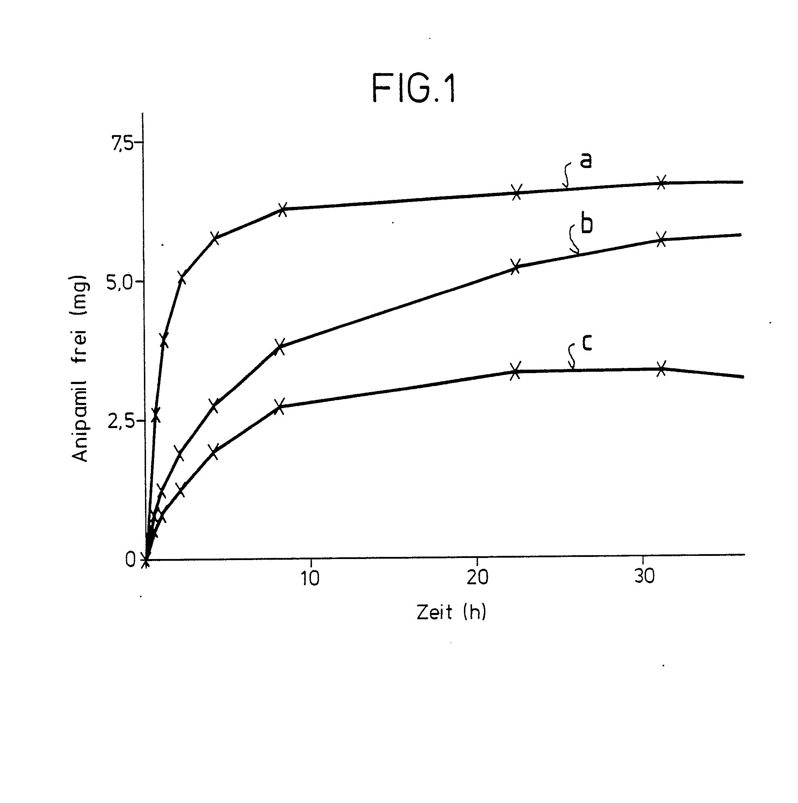

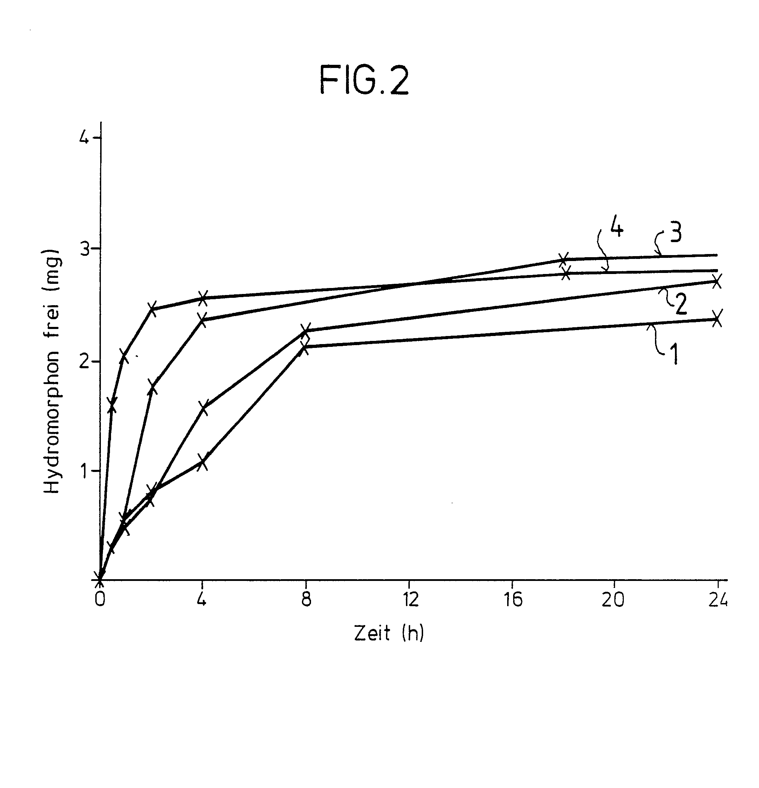

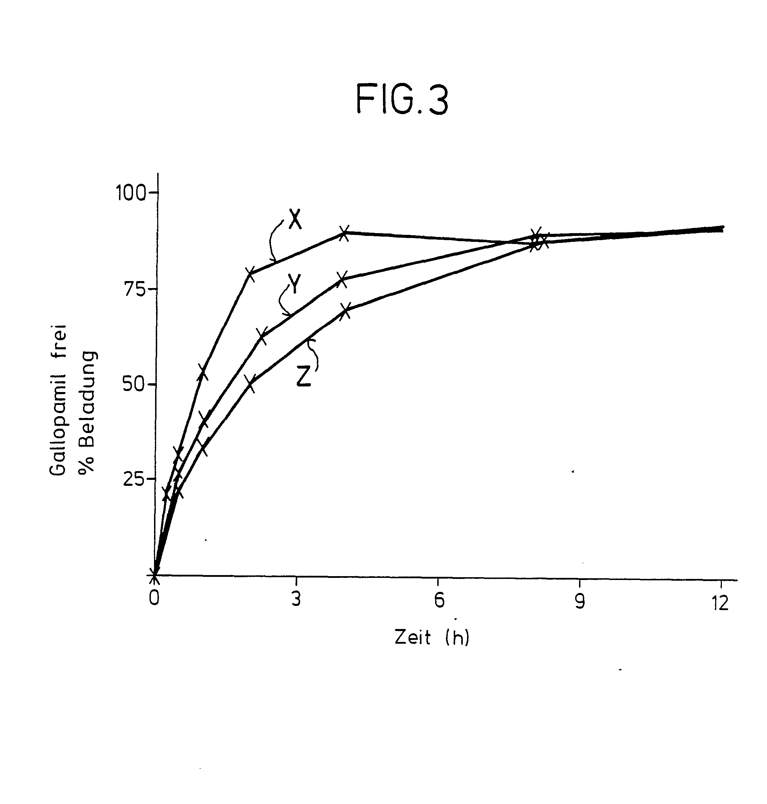

Eine Möglichkeit zur Steuerung der Freigabe ergibt sich durch den Einsatz von Dispersionen aus Polymeren und Copolymeren unterschiedlicher Polarität sowie unterschiedlichen Vernetzungsgrades. Durch Auswahl von Polymeren geeigneter Polarität kann die Bindung des Wirkstoffs im Polymer minimiert und somit die Freigabe des Wirkstoffs an die Haut optimiert werden. Ein Maß für die Polarität lipophiler Wirkstoffe und Polymerer stellt z.B. der Löslichkeitsparameter nach Hildebrandt, J.H., Prausnitz J.M., Scott R.L., Regular and Related Solutions, van Nostrand Reinhold, New York 1970, dar. Der Löslichkeitsparameter des Polymeren und das Ausmaß der Differenz der Löslichkeitsparameter zwischen Wirkstoff und Polymer können ein Maß für unterschiedliche Wechselwirkungen und damit für unterschiedliche Freigaberaten des Wirkstoffs aus dem Polymer sein. In Tabelle 1 und Figur 1 ist dies für den Wirkstoff Anipamil verdeutlicht. Auch über den Vernetzungsgrad des Polymeren kann die Freigaberate des Wirkstoffs gesteuert werden, wobei ein steigender Vernetzungsgrad die Löslichkeit des Wirkstoffs im Polymer vermindert und dadurch die Freigaberate steigert. Dies ist in Tabelle 2 und Figur 2 für den Wirkstoff Hydromorphon dargestellt. Ebenso ist eine Steuerung der Freisetzung über die Teilchengröße des Polymeren möglich, wobei kleinere Teilchen im allgemeinen eine schnellere Freisetzung bewirken. Dies ist in Tabelle 3 und Figur 3 für den Wirkstoff Gallopamil dargestellt. Eine weitere Möglichkeit zur Steuerung der Wirkstofffreigabe besteht in der Zumischung von lipophilen Hilfsstoffen (lipophiler als der Wirkstoff), beispielsweise von langkettigen Fettsäureestern wie Isopropylmyristat. Durch die Kombination aller vorgenannten Steuerungsprinzipien ist die Beeinflussung der Freigabe eines Wirkstoffs aus dem Wirkstoffträger in weiten Grenzen möglich.

Das erfindungsgemäß erhältliche Pflaster hat einen denkbar einfachen Aufbau, es besteht lediglich aus der Stütz- oder Trägerfolie und der wirkstoffhaltigen polymeren Klebeschicht. Es kann entweder in aufgerollter Form in den Handel kommen oder auf der Klebeschicht mit einer leicht abziehbaren Schutzfolie versehen sein. Das gesamte Herstellverfahren ist einfach und wirtschaftlich, weil in keiner Phase ein organisches flüchtiges Lösungsmittel verwendet wird, das später abgedampft werden müßte.

Die in den Beispielen genannten Teile und Prozente beziehen sich auf das Gewicht.


Beispiel 1

1 Teil Gallopamil-Base und 1 Teil Isopropylmyristat wurden unter Erwärmung auf 50 °C und starkem Rühren in 6 Teilen einer 1,5%igen wäßrigen Lösung des Natriumsalzes des Schwefelsäurehalbesters von 2 bis 3fach oxethyliertem $C_{12}$- bis $C_{14}$-Alkohol dispergiert. Diese Mischung wurde unter gleichen Bedingungen in 14 Teile einer 50%igen Poly-Butylacrylatdispersion eingerührt und die erhaltene wirkstoffhaltige Polymerdispersion auf einer glatten, ausgeloteten Unterlage mittels einer geeigneten Ausstreichrakel auf einer Aluminium-Polyethylen-Verbundfolie (Tscheulin GmbH, D-Teningen) ausgestrichen und bei 35-40 °C unter kontrollierter Luftfeuchtigkeit getrocknet. Der so hergestellte selbstklebende gallopamilhaltige Film mit einem Wirkstoffgehalt von 15% wurde dann in üblicher Weise weiter konfektioniert. Je nach Rakeleinstellung resultierten Flächengewichte von 5 mg/cm², die durch Mehrfachauftrag weiter erhöht werden konnten.


Beispiel 2

2 Teile Anipamil-Base wurden in einer Schmelze von 2 Teilen Hexadecan-(1)-ol bei 65 °C unter Rühren gelöst; diese Mischung wurde in 10 Teilen einer 2%igen wäßrigen Lösung des Natriumsalzes des Schwefelsäurehalbesters von 2 bis 3fach oxethyliertem $C_{12}$- bis $C_{14}$-Alkohol in Wasser unter starkem Rühren dispergiert. In die entstandene Emulsion wurden 30 Teile einer 50%igen wäßrigen Dispersion eines Copolymerisates aus 90 Gew.% 2-Ethylhexylacrylat, 7% Acrylnitril und 3% Acrylsäure bei gleichen Bedingungen langsam eingerührt und wie in Beispiel 1 beschrieben zu einem Film verarbeitet. Der so hergestellte selbstklebende Anipamil-haltige Film hatte einen Wirkstoffgehalt von 10%.


Beispiel 3

1 Teil Hydromorphon-HCl wurde in 10 Teilen einer 2%igen wäßrigen Lösung des Natriumsalzes des Schwefelsäurehalbesters von 2 bis 3fach oxethyliertem $C_{12}$- bis $C_{14}$-Alkohol gelöst. 100 Teile einer 50%igen sauren Dispersion eines Copolymerisats aus 50% Butylacrylat und 50% Vinylacetat wurden durch Zugabe von etwas weniger als 1 Teil konzentrierter $NH_3$-Lösung unter Rühren auf einen pH von 7,0 eingestellt, sodann mit 10 Teilen einer 7,5 %igen Lösung des oben und bei den Beispielen 1 und 2 bereits verwendeten anionischen Tensids gemischt. 10 Teile der emulgatorhaltigen Polymer-Dispersion wurden vorgelegt und 3 Teile der Hydromorphon-HCl-Lösung innerhalb 15 min bei Raumtemperatur eingerührt. Unter starkem Rühren (1000⁻¹) wurde dann durch tropfenweise Zugabe von 10% $NH_3$-Lösung innerhalb 15-30 min auf pH 9,5-10,0 gebracht, wobei Hydromorphon-Base freigesetzt wurde. Nach weiterem halbstündigen Rühren zur Homogenisierung der wirkstoffhaltigen Dispersion wurde diese wie in Beispiel 1 beschrieben zu einem Film verarbeitet. Der so hergestellte selbstklebende Hydromorphon-haltige Film hatte einen Wirkstoffgehalt von 5,5%.

4

Eigenschaften der wirkstoffhaltigen Polymerfilme

In-vitro-Freisetzung

Zur Bestimmung der in-vitro-Freisetzung wurden Filmstücke geeigneter Größe in eine Freigabezelle eingesetzt und bei festgelegter Temperatur und Durchmischung mit einem isotonischen Phosphatpuffer pH 6.0 als Akzeptormedium in Kontakt gebracht. Bei einzelnen Wirkstoffen wurde zur Erhöhung der Wirkstofflöslichkeit das Akzeptormedium mit 25% (V/V) Ethanol vermischt. Zu festgelegten Zeitpunkten wurden 3% des Freigabemediums als Probe gezogen und durch frisches Freigabemedium ersetzt. Der Wirkstoffgehalt der Proben wurde mittels HPLC bestimmt. Die Volumenkorrektur wurde nach dem Verdünnungsschema nach Münzel durchgeführt.

Einfluß der Polarität auf das Freigabeverhalten

Wirkstoffhaltige Filme mit Anipamil wurden aus 2 Latextypen unterschiedlicher Polarität gemäß Beispiel 2 hergestellt. Die aus den Freigabeuntersuchungen erhaltenen effektiven Diffusionskoeffizienten sind in Tabelle 1 dargestellt.

Tabelle 1

| Anipamil - effektiver Diffusionskoeffizient aus Polymerfilmen unterschiedlicher Polarität | | | |
|---|---|---|---|
| Polymerzusammensetzung | Löslichkeitsparameter $(kcal^{0,5} \cdot mol^{-1,5})$ | Wirkstoffkonzentration $(g \cdot cm^{-3})$ | Diffusionskoeffizient $(cm^2 \cdot s^{-1})$ |
| 49% Butadien, 49% Styrol, 2% Acrylsäure | 8,6 | 0,07272 | $6,188 \cdot 10^{-10}$ |
| 100% Butylacrylat | 9,5 | 0,07159 | $3,039 \cdot 10^{-9}$ |
| Löslichkeitsparameter Anipamil: $8,9 \ kcal^{0,5} \cdot mol^{-1,5}$ | | | |

In Figur 1 sind die Freigaberaten für die 2 Dispersionen
  a) Copolymerisat aus 49 gew.% Butadien, 49 Gew.% Styrol und 2 Gew.% Acrylsäure
  b) Polybutylacrylat
unterschiedlicher Polarität gezeigt. Wie aus den Kurvenverläufen ersichtlich, kann das Freigabeverhalten durch die Polarität des Polymeren beeinflußt werden.

Einfluß des Vernetzungsgrads auf das Freigabeverhalten

Wirkstoffhaltige Filme mit Hydromorphon wurden aus 3 Latextypen unterschiedlicher Vernetzung gemäß Beispiel 3 hergestellt. Die aus den Freigabeuntersuchungen erhaltenen effektiven Diffusionskoeffizienten sind in Tabelle 2 dargestellt.

Tabelle 2

| Hydromorphon - effektiver Diffusionskoeffizient in Polymerfilmen aus Poly-n-Butylacrylat unterschiedlichen Vernetzungsgrads | | |
|---|---|---|
| Polymervernetzungsgrad | Wirkstoffkonzentration $(g \cdot cm^{-3})$ | Diffusionskoeffizient $(cm^2 \cdot s^{-1})$ |
| unvernetzt, K = 78 | 0,0418 | $1,962 \cdot 10^{-10}$ |
| schwach vernetzt | 0,0417 | $4,506 \cdot 10^{-10}$ |
| stark vernetzt | 0,0434 | $2,302 \cdot 10^{-10}$ |
| Wirkstoffbeladung 3,0 ( +/- 0,1) mg pro 16 cm² Freigabefläche K = K-Wert nach H. Fikentscher, Cellulose-Chemie 13 (1932), S.58-64 und 71-74. | | |

In Figur 2 sind die Freigaberaten für 3 Dispersionen von Poly-n-butylacrylat unterschiedlichen Vernetzungsgrades dargestellt:

1) K-Wert 78
2) vernetzt mit 0,5 Gew.% Butandioldiacrylat
3) vernetzt mit 3 Gew.% Butandioldiacrylat

Wie aus den Kurvenläufen ersichtlich, kann das Freigabeverhalten des Wirkstoffs auch über den Vernetzungsgrad des Polymeren beeinflußt werden.

Einfluß der Latex-Teilchengröße auf das Freigabeverhalten

Wirkstoffhaltige Filme mit Gallopamil wurden aus drei Latextypen unterschiedlicher Latex-Teilchengröße gemäß Beispiel 1 hergestellt. Die aus den Freigabeuntersuchungen erhaltenen effektiven Diffusionskoeffizienten sind in Tabelle 3 dargestellt.

Tabelle 3

| Gallopamil - effektiver Diffusionskoeffizient in Polymerfilmen aus Poly-n-Butylacrylat unterschiedlicher Latex-Teilchengröße | | |
|---|---|---|
| Latex-Teilchengröße (nm) | Wirkstoffkonzentration (g $cm^{-3}$) | Diffusionskoeffizient $(cm^2 \ s^{-1})$ |
| 119 | 0,07877 | $2,726 \cdot 10^{-9}$ |
| 160 | 0,04900 | $1,772 \cdot 10^{-9}$ |
| 200 | 0,04795 | $1,263 \cdot 10^{-9}$ |

In Figur 3 sind die Freigaberaten für drei Dispersionen unterschiedlicher Latex-Teilchengröße dargestellt:

X) 119 nm
Y) 160 nm
Z) 200 nm.

Wie aus den Kurvenläufen ersichtlich, kann das Freigabeverhalten des Wirkstoffs auch über die unterschiedliche Latex-Teilchengröße des Polymeren beeinflußt werden.

Einfluß lipophiler Ester auf das Freigabeverhalten

Wirkstoffhaltige Filme mit Gallopamil wurden mit und ohne Isopropylmyristat als lipophilen Hilfsstoff gemäß Beispiel 1 hergestellt. Die aus den Freigabeuntersuchungen erhaltenen effektiven Diffusionskoeffizienten sind in der Tabelle 4 dargestellt.

Tabelle 4

| Gallopamil - effektiver Diffusionskoeffizient in Polymerfilmen aus Poly-n-Butylacrylat mit und ohne Isopropylmyristat (IPM) | | |
|---|---|---|
| IPM (Gew.%, bezogen auf Polymer) | Wirkstoffkonzentration $(g \cdot cm^{-3})$ | Diffusionskoeffizient $(cm^2 \cdot s^{-1})$ |
| - | 0,1535 | $5,782 \cdot 10^{-9}$ |
| 15 | 0,1513 | $12,175 \cdot 10^{-9}$ |

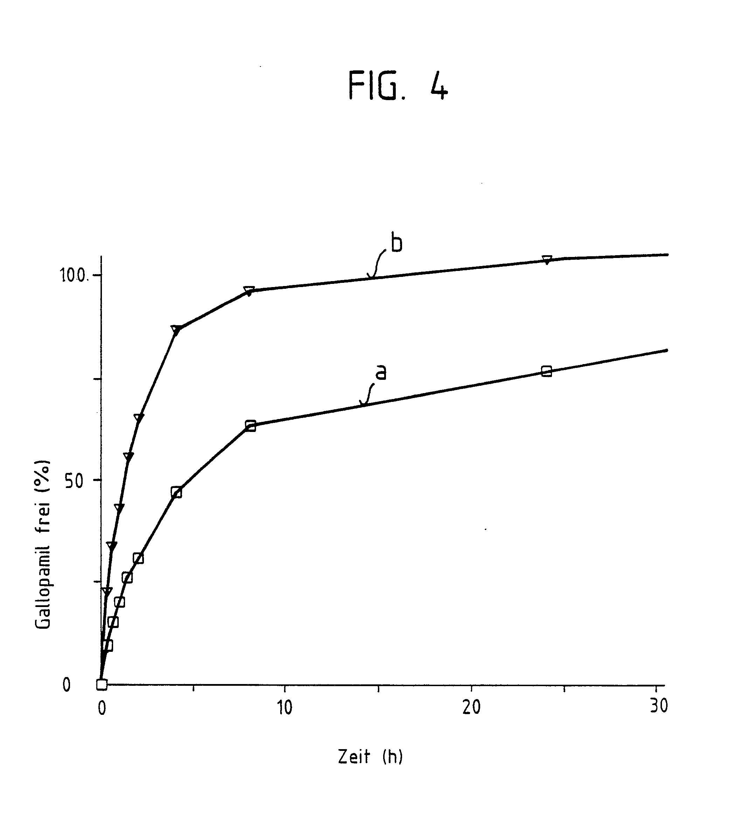

In Figur 4 sind die Freigaberaten für 2 Dispersionen
    a) Poly-n-Butylacrylat ohne IPM
    b) Poly-n-Butylacrylat mit 15 Gew.% IPM
dargestellt. Wie aus den Kurvenläufen ersichtlich, kann das Freigabeverhalten des Wirkstoffs auch über den Zusatz lipophiler Hilfsstoffe zum Polymer beeinflußt werden.

**Ansprüche**

1. Verfahren zur Herstellung eines Pflasters zur transdermalen Anwendung von systemisch wirksamen, lipophilen pharmazeutischen Wirkstoffen mit einem Molekulargewicht unter 1 000, durch Mischen einer wäßrigen Dispersion eines Polymeren mit einer Glastemperatur zwischen -70 und -10° C mit dem Wirkstoff, Beschichten einer Trägerfolie, die gegenüber Wasserdampf und dem Wirkstoff praktisch undurchlässig ist, mit der wirkstoffhaltigen Polymerdispersion und Trocknen, dadurch gekennzeichnet, daß ein Wirkstoff, der das Polymere nicht so stark anlöst, daß dessen Haftfähigkeit leidet, und kein flüchtiges organisches Lösungsmittel eingesetzt wird. .

2. Verfahren nach Anspruch 1, dadurch gekennzeichnet, daß man als Polymeres ein Butadien- oder (Meth-)Acrylestercopolymerisat mit 2 bis 8 Kohlenstoffatomen im Alkylrest einsetzt, das als Comonomere eines oder mehrere aus der Gruppe (Meth-)Acrylsäure, (Meth-)Acrylamid, (Meth-)Acrylnitril, Vinylacetat und Styrol einpolymerisiert enthält.

3. Verfahren nach Anspruch 1 oder 2, dadurch gekennzeichnet, daß das Polymer vernetzt ist.

4. Verfahren nach Anspruch 1 oder 2, dadurch gekennzeichnet, daß man einen Wirkstoff aus folgenden Gruppen einsetzt: Calciumantagonisten, Antihypertensiva, Antiarrhythmika, β-Rezeptorenblocker, starke Analgetika (Opiate), Psychopharmaka, Vasodilatatoren, Antiparkinsonmittel, Anticholinergika, Antihistaminika, Antirheumatika und Hormone.

# FIG.1

417/87

# FIG.2

# FIG.3

# FIG. 4

417/87